# EUROPEAN PATENT SPECIFICATION

(11) **EP 1 585 568 B1**
(45) Date of publication and mention of the grant of the patent: **19.02.2014**
(21) Application number: 03789544.8
(22) Date of filing: 17.12.2003
(51) Int. Cl.: A61M 5/32, A61M 5/20

(54) **INJECTION DEVICE**
INJEKTIONSVORRICHTUNG
DISPOSITIF D'INJECTION

(30) Priority: 17.12.2002 GB 0229404; 03.11.2003 GB 0325596
(43) Date of publication of application: 19.10.2005
(60) Divisional application: 10179742.1
(73) Proprietor: Cilag GmbH International, 6300 Zug (CH)
(72) Inventor: Harrison, Nigel David, Linton, Cambridge CB1 6YN (GB); Brady, Matthew James, Cambourne, Cambridgeshire CB3 6BU (GB); Berman, Gregory, Cambridge CB4 2AJ (GB); Johnston, David Maxwell, Lawrenceville, NJ 08648 (US); Habeshaw, Rosemary Louise, Cambridge CB1 7TS (GB)
(74) Representative: Tunstall, Christopher Stephen
(86) International application number: PCT/GB2003/005494
(87) International publication number: WO 2004/054645

(56) References cited:
- WO-A-95/29720
- WO-A-95/35126
- WO-A-03/092771
- GB-A- 2 388 033
- US-A- 6 099 504
- US-B1- 6 270 479

## Description

The present invention relates to an injection device, in particular an injection device which, having dispensed the contents of a syringe, automatically retracts the needle of the syringe.

Devices exist which are spring loaded to extend automatically the needle of a syringe from the device, dispense the contents of the syringe and then automatically retract the needle. WO 95/35126 and WO 95/29720 describe such devices.

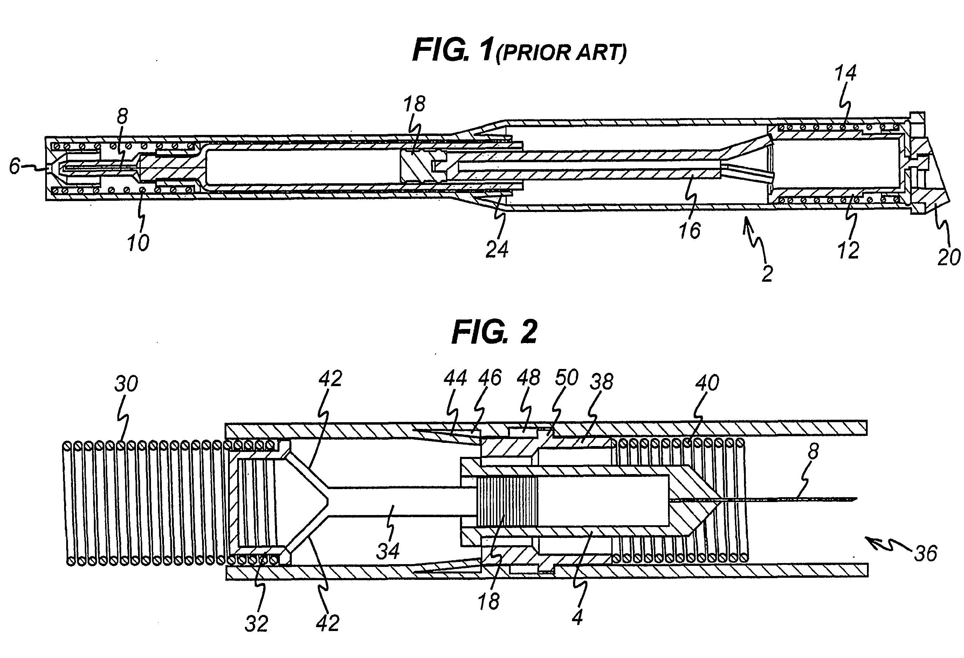

As illustrated in Figure 1 of the accompanying drawings, the device includes a housing 2 in which a syringe 4 is contained. The housing 2 includes an opening 6 through which the needle 8 of the syringe 4 may extend. A retraction spring 10 biases the syringe 4 away from the opening 6. The device also includes a drive element 12 which is biased by a spring 14 to drive a coupling 16 to move the dispensing piston 18 of the syringe 4. In use, a release mechanism 20 releases the drive element 12 such that the syringe 4 is first moved forwards and the needle 8 projects through the opening 6. Subsequently, the dispensing piston 18 is moved so as to expel the contents of the syringe 4. The device is designed to include a delatch mechanism. In particular, at the point at which the dispensing piston 18 reaches the end of the bore in the syringe 4, arms 22 at the end of the coupling 16 are deflected by a collar 24 within the housing 2 so as to disengage from the drive element 12. The arms 22 and coupling 16 may then move within a central passage of the drive element 12. As a result, by virtue of the bias of spring 10, the coupling 16 moves inside the drive element 12, the syringe 4 is driven away from the opening 6 and the needle 8 is retracted within the opening 6.

Other similar delatch or retract arrangements have also been proposed. For instance, EP-A-0 516 473 discloses one embodiment in which, at the point at which the dispensing piston reaches the end of the bore in the syringe, a portion of the coupling instantaneously collapses in length as the retraction spring retracts the needle of the spring.

In practice, all of these proposals suffer a problem that, owing to a stack up of tolerances of the various manufactured components of the assembled device (the dimensions of all manufacture components vary around a mean), it cannot be assured that the delatch mechanism will enable retraction of the syringe and needle at precisely the moment at which the dispensing piston reaches the end of the bore. In practice, either the mechanism delatches before the dispensing piston reaches the end of the bore, such that the syringe is not emptied, or the piston reaches the end of the bore before the mechanism has moved sufficiently far to delatch.

Although this problem has been recognised before, for instance in US 6,159,181, the proposed solution has been to provide a user actuated retraction mechanism rather than an automatic one. This is considered to be undesirable.

UK patent applications nos. 0210123 and 0229384 describe a series of injection devices designed to deal with this problem. In each case, the device includes a housing that receives a syringe and includes a resilient member that biases the syringe from an extended position to a retracted position. An actuator is used to advance the syringe from its retracted position to its extended position and discharge the contents of the syringe. The actuator includes a drive element and a drive coupling that transfers movement of the drive element to the syringe. The drive coupling is compressible and compression of the drive element begins once a release mechanism has been triggered. Release of the syringe takes place a little time after the release mechanism is triggered, in an attempt to ensure that the syringe has been completely discharged. The compressible drive coupling may be elastically or inelastically compressible.

Satisfactory though these injector devices are, the use of a compressible drive coupling has this result. Once the release mechanism has been triggered, actuator has to do work against the compressible drive coupling. In the case of an elastically compressible drive coupling, the actuator must to work to increase the elastic potential energy of the coupling. In the case of an elastically compressible drive coupling, the actuator must to work against the forces that resist its compression, such as fluid viscous forces. In each case, therefore, a proportion of the force that the actuator is capable of applying is diverted from the syringe to the compressible coupling, which means that the force applied to the syringe suddenly drops when the release mechanism is triggered. This may have adverse consequences.

The injection devices according to the present invention represent an improvement over those described in UK patent application publication nos. 2388033 and 2396298.

An injection device according to a first embodiment of the present invention comprises:
a housing adapted to receive a syringe having a discharge nozzle, the housing including means for biasing the syringe from an extended position in which the discharge nozzle extends from the housing to a retracted position in which the discharge nozzle is contained within the housing;
an actuator for exerting on one or more components of the syringe one or more forces that advance the syringe from its retracted position to its extended position and discharge the contents of the syringe through its discharge nozzle; and
a release mechanism, activated when one or more of the said components of the syringe have been advanced to one or more nominal release positions, adapted to release the syringe from the action of the actuator, whereupon the biasing means restores the syringe to its retracted position, the release of the syringe being delayed after such activation to allow continued exertion of the discharging force at substantially its magnitude immediately prior to such activation, to discharge any contents of the syringe remaining before the syringe is released.

The predetermined period of delay can be used to compensate for any stacking of tolerances. It becomes possible to ensure that the syringe has expelled its entire contents before it is retracted. It is not necessary for various components to be constructed with critical tolerances to ensure that the syringe is retracted just at the point at which its contents are fully dispensed.

Triggering of the release mechanism can be designed to occur before the contents of the syringe are fully dispensed. The predetermined delay is so designed that for all variations within the intended tolerances of the components, actual release of the syringe will not occur until after its contents have been fully dispensed.

The improvement represented by the present invention as compared with UK patent application publication nos. 2388033 and 2396298 is that, following activation of the release mechanism, the discharging force continues to be exerted at substantially its magnitude immediately beforehand.

The actuator comprises means for developing motion, and a coupling that is borne upon by a resilient element which acts as a motion-developing means and in turn bears upon the said one or more components of the syringe, thus exerting the said one or more advancing and discharging forces. The continued exertion of the discharging force at the relevant magnitude can be assured by providing for the distance between the point at which the motion-developing means bears upon the coupling and the point at which the coupling bears upon the said one or more components of the syringe to be substantially invariable. In this context, the term "motion-developing means" is intended to mean that component of the actuator which first develops motion and to exclude any downstream components that transfer the motion of that component to other components of the device. The motion developing means comprises a resilient member.

Invariance of the distance between the point at which the motion-developing means bears upon the coupling and the point at which the coupling bears upon the said one or more components of the syringe is most conveniently provided by a substantially incompressible coupling.

The biasing means may comprise a resilient member.

Preferably, the release mechanism is activated by the coupling reaching a predetermined position relative to the housing. In this way, the syringe can be secured within the housing at a position to ensure that the mechanism is triggered before its contents are fully dispensed.

Thus, the coupling advances and, at a predetermined position along the housing, the mechanism is triggered. This may be achieved by a feature of the coupling interacting with a trigger located on the inside of the housing.

Preferably, the release mechanism includes a resiliently biased release member for releasing the syringe, a trigger member for holding the release member and means for damping movement of the release member wherein, when the mechanism is activated, the trigger member releases the release member and the release member moves, under the influence of its resilient bias and against the resistance of the damping means, to release the syringe.

In this way, although the release member is biased to move to a position for releasing the syringe, the trigger member holds the release member back. Once the mechanism is triggered and the trigger member releases the release member, the release member still has to travel to the required position for releasing the syringe. By providing means for damping movement of the release member, movement of the release member is delayed such that the release of the syringe is also delayed.

Preferably, the damping means includes a fluid for damping the movement of the release member.

The damper could be provided on one or other of the release member and the housing with a pocket of fluid held in the other of the release member and housing.

Preferably, the coupling comprises first and second elements and the release mechanism is adapted to disengage the first and second coupling elements to release the syringe.

Once the first and second coupling elements are disengaged, one can move relative to the other. In particular, the second coupling element may be allowed to retract with the syringe.

Preferably, the first coupling element comprises an annular wall defining a central bore and the second coupling element includes resilient arms that engage the annular wall and are capable of inward deflection to disengage from the annular wall and allow the second coupling element to move relative to the first coupling element within the bore.

This provides a convenient and advantageous arrangement by which the first and second coupling elements can move relative to each other.

Inward deflection of the resilient arms may be achieved by means of a collar that is moveable within the housing. Movement of the collar allows it to be released from a first position upon activation of the release mechanism and move to a second position, in which the first and second coupling elements are disengaged.

Preferably, the damping means are provided between the collar and the housing. In this way, movement of the collar can be delayed to provide the required delay for release of the syringe.

Preferably, the trigger member is moveable by the coupling from a position to impede movement of the release member and a position to allow movement of the release member. Hence, the trigger member may take the form of a latch which physically impedes movement of the release member, for instance the collar. The first or second coupling element then physically moves the latch to enable the release member, to move and then subsequently release the syringe.

An alternative release mechanism includes an inertial mass moveable with the coupling and a release member actuated by the inertial mass to release the syringe, such that when the coupling reaches the said predetermined position relative to the housing, the inertial mass continues to move irrespective of movement of the coupling, so as to actuate the release member to release the syringe.

In this way, release of the syringe is not dependent on the relative positions of a number of different components and, hence, is not affected by tolerance stacking. Relative movement of the inertial mass and, hence, release of the syringe can occur as a direct result of the contents of the syringe being fully dispensed. It will be appreciated that the inertial mass has a momentum as it moves with the coupling towards the end of the housing. Once the contents of the syringe are fully dispensed, the coupling stops moving. The momentum of the inertial mass causes it to continue moving, and this movement can be used to actuate the release member to release the syringe.

Preferably, the inertial mass is mounted on the coupling so as to allow relative movement of the inertial mass in the direction of movement of the coupling during discharge of the syringe.

Preferably, the inertial mass is mounted on the coupling by means of a thread. In this way, rather than have the inertial mass move only with an axial or longitudinal movement relative to the coupling, the inertial mass can be caused additionally to spin or rotate. This allows a more controlled and steady momentum to be set up in the inertial mass and then used subsequently to release the syringe.

Preferably, the injection device further includes a stop that engages the inertial mass to prevent relative movement of the inertial mass in the direction opposite to the direction of movement of the coupling during discharge of the syringe .Preferably, the stop is provided on the coupling.

This is a convenient way of ensuring maximum transfer of momentum.

Preferably, the coupling comprises first and second elements and the release member is moveable by the inertial mass to disengage the first and second coupling elements to release the syringe.

The first coupling element may engage with the second in any convenient manner. For instance, one of them may include an annular wall defining a central bore and the other may include resilient arms that engage the annular wall and are capable of inward deflection to disengage from the annular wall and allow the two coupling elements to move relative to each other. According to the type of engagement chosen, the release member may take any suitable form. For instance, for the example described here, the release member could include a moveable collar acting on the arms to deflect them inwardly.
the release member is integral with the inertial mass and connects the first and second coupling elements, such that relative movement of the inertial mass and the coupling separates the first and second coupling elements to release the syringe.

The release member may be integral with the inertial mass and connect the two coupling elements, such that relative movement of the inertial mass and the coupling separates the two coupling elements to release the syringe.

In other words, the inertial mass may itself form part of a component which joins one coupling element to the other. Once the syringe has been fully discharged and the inertial mass moves relative to the coupling, it can be arranged to release the connection between the two coupling elements, allowing retraction of the syringe.

Preferably, the two coupling elements are provided with coaxial threads and the release member is provided with a corresponding thread to connect the coupling elements.

In this way, when the syringe is fully discharged, the momentum of the inertial mass will cause it to rotate on its thread relative to the coupling, thereby releasing the connection between the two coupling elements and allowing retraction of the syringe.

Alternatively, the release member may be moveable to disengage the motion-developing means from the coupling. This may be achieved in the same manner as described above for the two coupling elements.

Where the release member is not an integral member of the inertial mass, it may be provided on an inner part of the housing.

The invention will be more clearly understood from the following description given by way of example only, with reference to the accompanying drawings in which:
Figure 1 illustrates a known construction for an injection device;
Figures 2 to 7 illustrate a first embodiment of the present invention at different stages of its operation;
Figures 8 to 10 illustrate a second embodiment of the present invention at different stages of its operation; and
Figure 11 illustrates a variation on the second embodiment.

The present invention can be embodied in any suitable outer housing such as illustrated in Figure 1, in particular having an opening 6 in its end through which the needle 8 of a syringe 4 may extend and a release mechanism 20 at its opposite end for releasing a spring 14 for deploying and emptying the contained syringe.

Figure 2 illustrates schematically the key components of a preferred embodiment for use in a preferred housing.

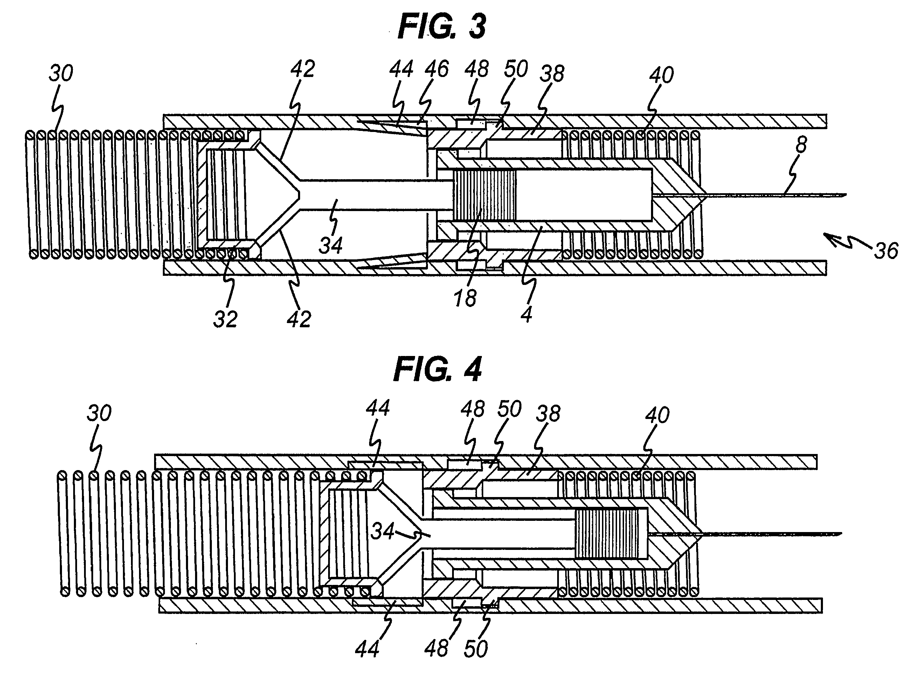

A drive spring 30 engages with a first coupling element 32, itself providing drive to a second coupling element 34. Thus, the drive spring 30 here acts as the motion-developing means for the injection device. Alternative motion-developing means could be used, for example a pneumatic piston operated by a compressed gas canister or the actuator of a solenoid in an electrically powered device. The second coupling element 34 is provided to engage the dispensing piston 18 of a syringe 4 in the device. Thus, when the coupling elements 32, 34 are released by an appropriate release mechanism, the drive spring 30 drives the first coupling element 32 and, hence, the second coupling element 34 towards an end 36 of the housing. As is known, when the second coupling element 34 first pushes upon the dispensing piston 18 of the syringe 4, it will move the syringe 4 itself towards the end 36 of the housing as illustrated in Figure 3. Indeed, the syringe 4 will be moved such that its needle 8 protrudes from the end 36 of the housing so as to penetrate the skin of a user.

As illustrated in Figure 4, further movement of the first coupling element 32 and second coupling element 34 will cause the dispensing piston 18 to move relative to the cylindrical housing of the syringe 4 and thereby expel the contents of the syringe 4 through the needle 8.

The injection device also includes a mechanism for retracting the syringe 4. Although not illustrated, a spring may be provided to bias the syringe 4 inwardly of the housing and away from the end 36. Once the dispensing piston 18 has reached the end of its travel in the syringe 4, the mechanism may release the syringe such that the spring moves the syringe 4 inwardly of the housing, thereby retracting the needle 8 from the user.

According to the illustrated embodiment, the mechanism includes a release member in the form of a collar 38. The collar 38 is moveable axially within the housing and is biased away from the end 36 of the housing by means of a spring 40. In this embodiment, collar 38 is designed to interact with resilient arms 42 provided on an end of the second coupling element 34 engaging the first coupling element 32. The resilient arms 42 interconnect the first coupling element 32 and second coupling element 34 such that the first coupling element 32 may push against and transfer movement to the second coupling element 34.

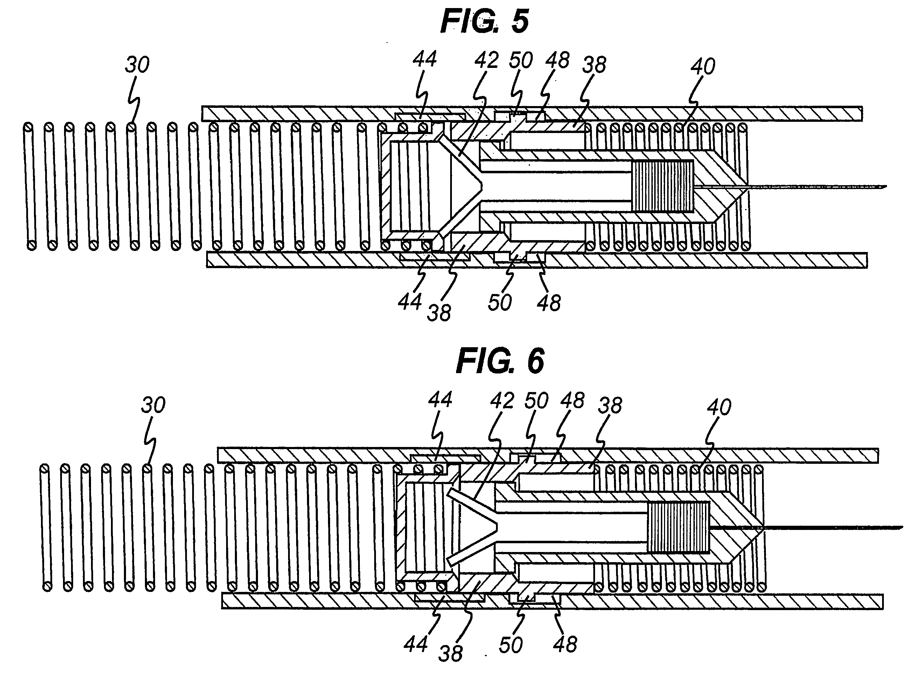

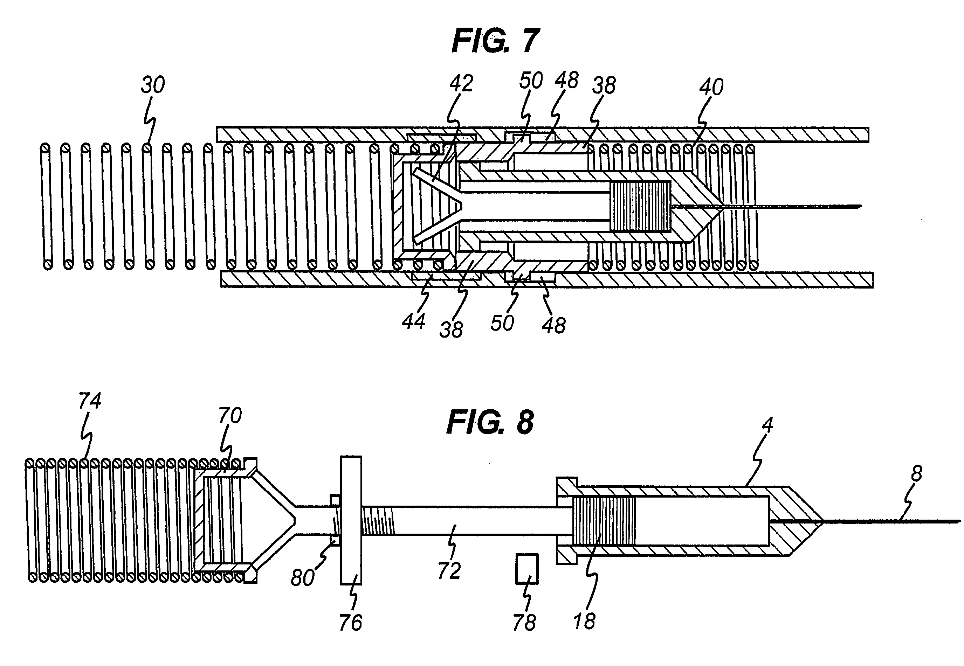

As illustrated in Figure 5, the collar 38 is able to push upon the resilient arms 42 and deflect them inwardly. In this way, as illustrated in Figure 6, the resilient arms 42 are pushed inwardly into a through hole defined within the first coupling element 32. The syringe 4 is thereby released and can travel as illustrated in Figure 7 so as to retract the needle 8.

According to the present invention, the syringe is not released until a predetermined period after the mechanism is triggered. In this respect, it will be seen in the Figures that at least one trigger member 44 is provided so as to prevent the collar 38 moving by virtue of the spring 40. Hence, in Figures 2 and 3, the collar 38 is held in a position towards the end 36 of the housing. The trigger member(s) 44 are provided adjacent corresponding recesses 46 into which they may be deflected.

As the first coupling element 32 and second coupling element 34 move towards the end 36 of the housing, at some predetermined position, they deflect the trigger member(s) 44 so as to trigger the mechanism. As illustrated, the trigger member(s) 44 is provided at the outer periphery of the path of the first coupling element 32. Hence, for this embodiment, the first coupling element 32 itself deflects the trigger member(s) outwardly. However, it should be appreciated that other similar trigger members could be provided which are deflected either by the first coupling element 32 or the second coupling element 34.

As illustrated in Figure 4, with the trigger member(s) deflected into the corresponding recess(es) 46, the collar 38 is free to move away from the end 36 of the housing under the power of spring 40 so as to deflect the resilient arms 42 and release the syringe 4 as described above.

It will be appreciated that there will be a predetermined time interval between deflection of the trigger member(s) 44 and deflection of the resilient arms 42 due to the time taken for the collar 38 to move. During this predetermined time interval, the first coupling element 32 and second coupling element 34 will continue to move the dispensing piston 18 towards the end surface of the syringe 4. Indeed, in the preferred embodiment, the components are designed such that the trigger member(s) 44 are deflected and the mechanism is triggered before the dispensing piston 18 reaches the end surface of the syringe 4 (see Figure 4), but that the syringe 4 is not released until after the dispensing piston 18 reaches the end surface of the syringe 4. In this embodiment, this is when the resilient arms 42 are deflected (see Figures 5 and 6). It will be noted that, throughout the period of time taken by the collar 38 to collapse the resilient arms 42, the first coupling element 32 continues to bear directly upon the resilient arms 42 of the second coupling element 34. Thus, substantially all of the resilient force of the drive spring 30 is transferred, via the first and second coupling elements 32, 34, to the dispensing piston of the syringe. Substantially no work is done by the drive spring 30 on the collar 38, because the point contacts of the collar 38 on the resilient arms 42 are substantially frictionless.

Although it is possible to rely on frictional forces to resist movement of the collar 38 and thereby introduce the desired delay, in the preferred embodiment, a damping arrangement is provided.

As illustrated in Figures 2 to 7, the housing is provided with a damping pocket 48 within which a damper 50 on the outer periphery of the collar 38 may move. In this respect, it should be appreciated that the arrangement of pocket and damper between the housing and collar can be reversed.

The pocket 48 may be filled with a damping fluid, such as a liquid, or may rely merely on movement of a gas, such as air. Once the collar 38 is released by the trigger member(s) 44, the damping arrangement, 48, 50 resists movement and thereby provides an increased delay before the syringe 4 is released. As explained above, this can be used to ensure that the dispensing piston 18 has fully expelled the contents of the syringe 4 before the syringe 4 is released.

While a particular embodiment has been described, it should be appreciated that a large number of variations may be introduced. It is not necessary for the release member to be a collar or, indeed, the collar 38 might rotate instead of or as well as move axially. Furthermore, any other form of damping suitable for the release member may be used and, as mentioned above, different forms of trigger member are also possible.

Figures 8, 9 and 10 illustrate schematically key components of an alternative embodiment. Once again, these components can be embodied in an overall housing such as illustrated in Figure 1 with an equivalent release mechanism. Also, many of the features described with reference to Figures 2 to 7 could also be used with this embodiment to disconnect the drive spring from the first coupling element, or the first coupling element from the second coupling element so as to enable retraction of the syringe under the power of a retraction spring.

As with previous arrangements, a first coupling element 70 engages with a second coupling element 72 which in turn engages with the dispensing piston 18 of a syringe 4. A drive spring 74 may be provided to move the syringe 4 towards an end of the housing so as to extend the needle 8 of the syringe 4 and subsequently to move the dispensing piston 18 within the syringe 4 so as to expel the contents of the syringe 4. Alternatively, other motion-developing means may be used.

Although not illustrated, a resilient member such as a spring may also be provided to bias the syringe and needle inwardly of the housing such that, at an appropriate time, the syringe 4 and needle 8 may be retracted back into the housing.

A mechanism is provided whereby the syringe 4 is released at an appropriate time so that the resilient member can retract the syringe 4. Details of the components which hold the syringe and release it are not essential to the invention. Components such as described above and other known mechanisms may be used. However, the present invention proposes a novel and inventive arrangement for triggering retraction of the syringe 4.

In the embodiment illustrated in Figures 8 to 10, an inertial mass 76 is mounted on the second coupling element 72.

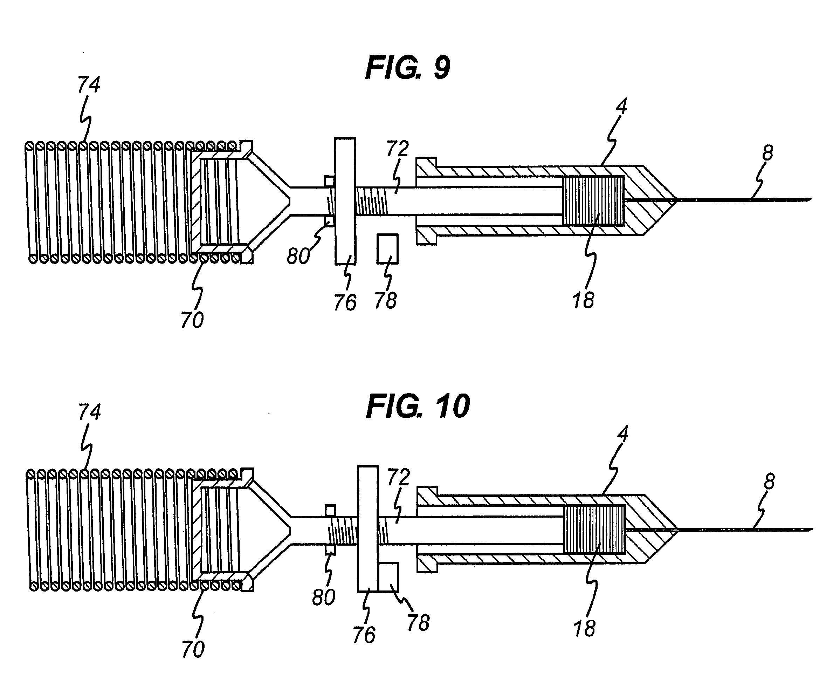

When the first coupling element 70 and second coupling element 72 move forwards so as to move the dispensing piston 18 and expel the contents of the syringe 4, the inertial mass 76 moves with them. However, when the dispensing piston 18 reaches the internal end surface of the syringe 4 as illustrated in Figure 9, the first coupling element 70 and second coupling element 72 stop moving relatively abruptly. Due to the momentum of the inertial mass 76, the inertial mass 76 continues to move forwards.

As illustrated, a trigger 78 is provided in the device for actuating the retraction mechanism. In particular, the inertial mass 76 will move from its original position on the second coupling element 72 to a position where it will operate the trigger 78 and thereby actuate the retraction mechanism. It will be noted that, throughout the period of time taken by the inertial mass 76 to reach the trigger 78, the first coupling element 70 continues to bear directly upon the second coupling element 72. Thus, substantially all of the resilient force of the drive spring 74 is transferred, via the first and second coupling elements 70, 72, to the dispensing piston of the syringe.

It will be appreciated that a substantial number of variations are possible. The inertial mass could be mounted on the first coupling element 70 or, indeed, it could be mounted on a separate component which also moves with the dispensing piston 18. The important point is of course that the inertial mass should move to actuate the retraction mechanism in response to the dispensing piston 18 stopping.

Similarly, the trigger 78 can take any appropriate form and can be mounted anywhere within the housing, for instance on the housing wall or on the second coupling element 72. The nature of the trigger 78 may vary according to the particular retraction mechanism used.

In the illustrated embodiment, a stop 80 is provided behind the inertial mass 76. The stop 80 functions to push the inertial mass 76 forwards when the first coupling element 70 and second coupling element 72 first start moving forwards so as to move the syringe 4 and dispensing piston 18. It will be appreciated that, at this point in the operation of the device, there will be a tendency for the inertial mass 76 to move backwards away from the dispensing piston 18 due to its inertia. By providing the stop 80, the inertial mass 76 is positively driven forwards with the second coupling element 72 such that the energy and momentum provided to the inertial mass 76 is maximised. This similarly maximises its ability to operate the trigger 78.

The stop 80 may be a separate component or be formed as an integral part of the mounting for the inertial mass. For instance, where the inertial mass is able to move relative to the second coupling element along a channel, the channel might only start at a predetermined position along the length of the second coupling element 72 such that the end wall of the channel forms the stop.

In the preferred embodiment, the inertial mass 76 is mounted rotatably by means of a thread. In the illustrated embodiment, the inertial mass 76 may have a female thread engaging with a corresponding male thread around the second coupling element 72. However, as mentioned above, the inertial mass 76 could alternatively be mounted on other components.

In this way, when the dispensing piston 18 reaches the end of its travel and the inertial mass 76 starts to move relative to the second coupling element 72, the inertial mass will rotate or spin as well as move forwards towards the dispensing piston 18. This rotation provides a rotational inertia or momentum which is more controlled and sustains over a longer period of time. In particular, it can be more effective in providing assured actuation of any trigger.

In alternative embodiments, the inertial mass and release member may be integrated so as to themselves form the retraction mechanism. In particular, the release member may take the form of a thread which joins the first coupling element to the second coupling element.

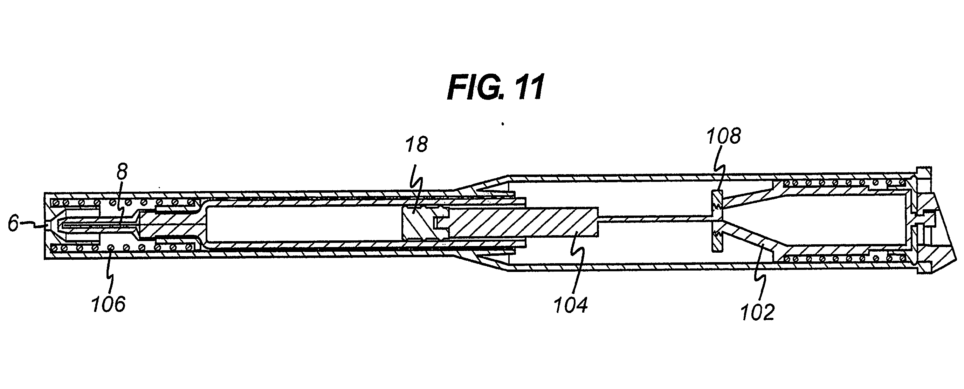

An embodiment is illustrated in Figure 11.

When the dispensing piston 18 reaches the end of its travel and stops, the inertial mass 108 continues to move and hence rotates about the first coupling element 102 and second coupling element 104 such that its thread forming the release member unscrews from the threads joining the first coupling element 102 and the second coupling element 104. Once the thread has fully unscrewed then second coupling element 104 is able to move relative to the first coupling element 102 and the syringe 4 is retracted by means of the resilient member 106. Throughout the period of time taken by the inertial mass 108 to unscrew from the threads on the second coupling element 104, the first coupling element 102 continues to bear upon the second coupling element 104 via the inertial mass 108. Thus, substantially all of the drive force transferred to the dispensing piston of the syringe.

## Claims

1. An injection device comprising:
a housing adapted to receive a syringe (4) having a discharge nozzle, the housing including means (40) for biasing the syringe (4) from an extended position in which the discharge nozzle extends from the housing to a retracted position in which the discharge nozzle is contained within the housing;
an actuator (30) for exerting on one or more components of the syringe (4) one or more forces that advance the syringe (4) from its retracted position to its extended position and discharge the contents of the syringe (4) through its discharge nozzle, the actuator (30) comprising means for developing motion (30) of the syringe via a coupling (32, 34) that is borne upon by the motion-developing means (30) and in turn bears upon the said one or more components of the syringe (4), thus exerting the said one or more advancing and discharging forces; and
a release mechanism, activated when one or more of the said components of the syringe (4) have been advanced to one or more nominal release positions, adapted to release the syringe (4) from the action of the actuator (30), whereupon the biasing means (40) restores the syringe (4) to its retracted position;
the release mechanism is adapted to delay the release of the syringe after activation of the release mechanism to allow continued exertion of the discharging force at substantially its magnitude immediately prior to such activation, to discharge any contents of the syringe (4) remaining before the syringe (4) is released **characterised in that**
the motion-developing means (30) comprises a resilient member.

2. An injection device according to claim 1 in which the distance between the point at which the motion-developing means (30) bears upon the coupling (32, 34) and the point at which the coupling (32, 34) bears upon the said one or more components of the syringe (4) is substantially invariable.

3. An injection device according to claim 2 in which the coupling (32, 34) is substantially incompressible.

4. An injection device according to any preceding claim in which the biasing means (40) comprises a resilient member.

5. An injection device according to any preceding claim in which the release mechanism is activated by the coupling (32, 34) reaching a predetermined position relative to the housing.

6. An injection device according to claim 5 in which the release mechanism includes:
a resiliently biased release member (38) for releasing the syringe (4);
a trigger member (44) for holding the release member (38); and
means for damping movement of the release member (38);
in which, when the mechanism is activated, the trigger member (44) releases the release member (38) and the release member (38) moves, under the influence of its resilient bias and against the resistance of the damping means, to release the syringe (4).

7. An injection device according to claim 6 in which the damping means includes a fluid for damping the movement of the release member (38).

8. An injection device according to claim 7 in which the damping fluid is a liquid.

9. An injection device according to any one of claims 6-8 in which the release member (38) includes a collar.

10. An injection device according to claim 9 in which the damping means is provided between the collar (38) and the housing.

11. An injection device according to any one of claims 6-10 in which the trigger member (44) is moveable by the coupling (32, 34) from a position that impedes movement of the release member (38) to a position that allows movement of the release member (38).

12. An injection device according to any preceding claim in which the coupling comprises first (32) and second (34) elements and the release mechanism (38) is adapted to disengage the first and second elements (32, 34) to release the syringe (4).

13. An injection device according to claim 12 in which the first element (32) comprises an annular wall defining a central bore and the second element includes resilient arms (42) that engage the annular wall and are capable of inward deflection to disengage from the annular wall and allow the second element (34) to move relative to the first element (32) within the bore.

14. An injection device according to claim 5, in which:
the release mechanism includes an inertial mass (76) moveable with the coupling (70, 72) and a release member actuated by the inertial mass (76) to release the syringe (4), such that when the coupling (70, 72) reaches the said predetermined position relative to the housing, the inertial mass continues to move irrespective of movement of the coupling (70, 72), so as to actuate the release member to release the syringe (4).

15. An injection device according to claim 14 in which the inertial mass (76) is mounted on the coupling (70, 72) so as to allow relative movement of the inertial mass (76) in the direction of movement of the coupling (70, 72) during discharge of the syringe (4).

16. An injection device according to claim 15 in which the inertial mass (76) is mounted on the coupling (70, 72) by means of a thread.

17. An injection device according to claims 15 or claim 16, further including a stop (80) that engages the inertial mass to prevent relative movement of the inertial mass (76) in the direction opposite to the direction of movement of the coupling (70, 72) during discharge of the syringe (4).

18. An injection device according to claim 17 in which the stop (80) is provided on the coupling (70, 72).

19. An injection device according to any one of claims 14 to 18 in which the coupling (70, 72) comprises first (70) and second (72) elements and the release member is moveable by the inertial mass (76) to disengage the first (70) and second (72) elements to release the syringe (4).

20. An injection device according to claim 19 in which the release member is integral with the inertial mass (76) and connects the first (70) and second (72) elements, such that relative movement of the inertial mass (76) and the coupling (70, 72) separates the first (70) and second (72) elements to release the syringe (74).

21. An injection device according to claim 20 in which the first (70) and second (72) elements are provided with coaxial threads and the release member is provided with a corresponding thread to connect the first and second coupling elements (70, 72).

## Patentansprüche

1. Injektionsvorrichtung, umfassend:
ein Gehäuse, das geeignet ist, eine Spritze (4) mit einer Austragdüse aufzunehmen, wobei das Gehäuse ein Mittel (40) zum Vorspannen der Spritze (4) aus einer ausgefahrenen Position, in der die Austragdüse aus dem Gehäuse ausgefahren ist, in eine zurückgezogene Position, in der die Austragdüse im Gehäuse enthalten ist, aufweist,
ein Stellglied (30) zum Ausüben einer oder mehrerer Kräfte auf eine oder mehrere Komponenten der Spritze (4), die die Spritze (4) aus ihrer zurückgezogenen Position in ihre ausgefahrene Position vorschieben und den Inhalt der Spritze (4) durch ihre Austragdüse austragen, wobei das Stellglied (30) ein Mittel (30) zur Entwicklung der Bewegung der Spritze über eine Kupplung (32, 34) umfasst,
die von dem Bewegungsentwicklungsmittel (30) beaufschlagt wird und ihrerseits die eine oder mehreren Komponenten der Spritze (4) beaufschlagt, so dass die eine oder mehreren Vorschub- und Austragskräfte ausgeübt werden, und
einen Freigabemechanismus, der aktiviert wird, wenn eine oder mehrere der Komponenten der Spritze (4) in eine oder mehrere Nennfreigabepositionen vorgeschoben worden sind, und geeignet ist, die Spritze (4) von der Wirkung des Stellglieds (30) freizugeben, woraufhin das Vorspannmittel (40) die Spritze (4) in ihre zurückgezogene Position zurückstellt, wobei der Freigabemechanismus geeignet ist, die Freigabe der Spritze nach Aktivierung des Freigabemechanismus zu verzögern, um eine fortgesetzte Ausübung der Austragskraft in im Wesentlichen der Höhe unmittelbar vor einer derartigen Aktivierung zu gestatten, um etwaigen noch verbleibenden Inhalt der Spritze (4) auszutragen, bevor die Spritze (4) freigegeben wird, **dadurch gekennzeichnet, dass**
das Bewegungsentwicklungsmittel (30) ein elastisches Element umfasst.

2. Injektionsvorrichtung nach Anspruch 1, wobei der Abstand zwischen der Stelle, an der das Bewegungsentwicklungsmittel (30) die Kupplung (32, 34) beaufschlagt, und der Stelle, an der die Kupplung (32, 34) die eine oder mehreren Komponenten der Spritze (4) beaufschlagt, im Wesentlichen unveränderlich ist.

3. Injektionsvorrichtung nach Anspruch 2, wobei die Kupplung (32, 34) im Wesentlichen nicht zusammendrückbar ist.

4. Injektionsvorrichtung nach einem der vorhergehenden Ansprüche, wobei das Vorspannmittel (40) ein elastisches Element umfasst.

5. Injektionsvorrichtung nach einem der vorhergehenden Ansprüche, wobei der Freigabemechanismus dadurch aktiviert wird, dass die Kupplung (32, 34) eine vorbestimmte Position bezüglich des Gehäuses erreicht.

6. Injektionsvorrichtung nach Anspruch 5, wobei der Freigabemechanismus Folgendes aufweist:
ein elastisch vorgespanntes Freigabeelement (38) zur Freigabe der Spritze (4),
ein Auslöseelement (44) zum Halten des Freigabeelements (38) und
ein Mittel zum Dämpfen der Bewegung des Freigabeelements (38),
wobei das Auslöseelement (44) das Freigabeelement (38) freigibt und sich das Freigabeelement (38) unter dem Einfluss seiner elastischen Vorspannung und gegen den Widerstand des Dämpfmittels bewegt, um die Spritze (4) freizugeben, wenn der Mechanismus aktiviert wird.

7. Injektionsvorrichtung nach Anspruch 6, wobei das Dämpfmittel ein Fluid zum Dämpfen der Bewegung des Freigabeelements (38) aufweist.

8. Injektionsvorrichtung nach Anspruch 7, wobei das Dämpffluid eine Flüssigkeit ist.

9. Injektionsvorrichtung nach einem der Ansprüche 6 - 8, wobei das Freigabeelement (38) einen Bund aufweist.

10. Injektionsvorrichtung nach Anspruch 9, wobei das Dämpfmittel zwischen dem Bund (38) und dem Gehäuse vorgesehen ist.

11. Injektionsvorrichtung nach einem der Ansprüche 6 - 10, wobei das Auslöseelement (44) durch die Kupplung (32, 34) aus einer Position, die die Bewegung des Freigabeelements (38) behindert, in eine Position, die die Bewegung des Freigabeelements (38) gestattet, bewegt werden kann.

12. Injektionsvorrichtung nach einem der vorhergehenden Ansprüche, wobei die Kupplung ein erstes (32) und ein zweites (34) Element umfasst und der Freigabemechanismus (38) geeignet ist, das erste und das zweite Element (32, 34) auszurücken, um die Spritze (4) freizugeben.

13. Injektionsvorrichtung nach Anspruch 12, wobei das erste Element (32) eine ringförmige Wand umfasst, die eine mittlere Bohrung definiert, und das zweite Element elastische Arme (42) aufweist, die die ringförmige Wand in Eingriff nehmen und sich nach innen durchbiegen können, um aus der ringförmigen Wand auszurücken und dem zweiten Element (34) zu gestatten, sich in der Bohrung bezüglich des ersten Elements (32) zu bewegen.

14. Injektionsvorrichtung nach Anspruch 5, wobei der Freigabemechanismus eine Inertialmasse (76), die sich mit der Kupplung (70, 72) bewegen kann, und ein Freigabeelement aufweist, das durch die Inertialmasse (76) betätigt wird, um die Spritze (4) freizugeben, so dass sich die Inertialmasse, wenn die Kupplung (70, 72) die vorbestimmte Position bezüglich des Gehäuses erreicht, unabhängig von der Bewegung der Kupplung (70, 72) weiter bewegt, um das Freigabeelement zur Freigabe der Spritze (4) zu betätigen.

15. Injektionsvorrichtung nach Anspruch 14, wobei die Inertialmasse (76) an der Kupplung (70, 72) montiert ist, um eine Relativbewegung der Inertialmasse (76) in der Bewegungsrichtung der Kupplung (70, 72) während des Austragens der Spritze (4) zu gestatten.

16. Injektionsvorrichtung nach Anspruch 15, wobei die Inertialmasse (76) mittels eines Gewindes an der Kupplung (70, 72) montiert ist.

17. Injektionsvorrichtung nach Anspruch 15 oder 16, die ferner einen Anschlag (80) aufweist, der die Inertialmasse in Eingriff nimmt, um eine Relativbewegung der Inertialmasse (76) in der der Bewegungsrichtung der Kupplung (70, 72) entgegengesetzten Richtung während des Austragens der Spritze (4) zu verhindern.

18. Injektionsvorrichtung nach Anspruch 17, wobei der Anschlag (80) an der Kupplung (70, 72) vorgesehen ist.

19. Injektionsvorrichtung nach einem der Ansprüche 14 bis 18, wobei die Kupplung (70, 72) ein erstes (70) und ein zweites (72) Element umfasst und das Freigabeelement durch die Inertialmasse (76) bewegt werden kann, um das erste (70) und das zweite (72) Element zur Freigabe der Spritze (4) auszurücken.

20. Injektionsvorrichtung nach Anspruch 19, wobei das Freigabeelement mit der Inertialmasse (76) integral ist und das erste (70) und das zweite (72) Element verbindet, so dass das erste (70) und das zweite (72) Element zur Freigabe der Spritze (74) durch die Relativbewegung der Inertialmasse (76) und der Kupplung (70, 72) getrennt werden.

21. Injektionsvorrichtung nach Anspruch 20, wobei das erste (70) und das zweite (72) Element mit koaxialen Gewinden versehen sind und das Freigabeelement mit einem entsprechenden Gewinde versehen ist, um das erste und das zweite Kupplungselement (70, 72) zu verbinden.

## Revendications

1. Dispositif d'injection comprenant :
un boîtier prévu pour recevoir une seringue (4) ayant une buse de décharge, le boîtier comportant un moyen (40) pour solliciter la seringue (4) d'une position sortie dans laquelle la buse de décharge s'étend hors du boîtier dans une position rentrée dans laquelle la buse de décharge est contenue à l'intérieur du boîtier ;
un actionneur (30) pour exercer, sur un ou plusieurs composants de la seringue (4), une ou plusieurs forces qui font avancer la seringue (4) de sa position rentrée dans sa position sortie et qui décharge le contenu de la seringue (4) à travers sa buse de décharge, l'actionneur (30) comprenant un moyen pour développer le mouvement (30) de la seringue par le biais d'un accouplement (32, 34) contre lequel s'appuie le moyen de développement de mouvement (30) et qui s'appuie à son tour sur lesdits un ou plusieurs composants de la seringue (4), en exerçant ainsi lesdites une ou plusieurs forces d'avance et de décharge ; et
un mécanisme de libération, activé lorsqu'un ou plusieurs desdits composants de la seringue (4) ont été avancés jusqu'à une ou plusieurs positions de libération nominales, prévu pour libérer la seringue (4) de l'action de l'actionneur (30), le moyen de sollicitation (40) ramenant la seringue (4) dans sa position rentrée ;
le mécanisme de libération étant prévu pour retarder la libération de la seringue après l'activation du mécanisme de libération afin de permettre de continuer à exercer la force de décharge substantiellement à l'ampleur qu'elle avait immédiatement avant une telle activation, afin de décharger le contenu de la seringue (4) éventuellement restant avant la libération de la seringue (4), **caractérisé en ce que** le moyen de développement de mouvement (30) comprend un organe élastique.

2. Dispositif d'injection selon la revendication 1, dans lequel la distance entre le point au niveau duquel le moyen de développement de mouvement (30) s'appuie sur l'accouplement (32, 34) et le point au niveau duquel l'accouplement (32, 34) s'appuie sur lesdits un ou plusieurs composants de la seringue (4) est substantiellement invariable.

3. Dispositif d'injection selon la revendication 2, **caractérisé en ce que** l'accouplement (32, 34) est substantiellement incompressible.

4. Dispositif d'injection selon l'une quelconque des revendications précédentes, dans lequel le moyen de sollicitation (40) comprend un organe élastique.

5. Dispositif d'injection selon l'une quelconque des revendications précédentes, dans lequel le mécanisme de libération est activé par l'accouplement (32, 34) atteignant une position prédéterminée par rapport au boîtier.

6. Dispositif d'injection selon la revendication 5, dans lequel le mécanisme de libération comporte :
un organe de libération sollicité de manière élastique (38) pour libérer la seringue (4) ;
un organe de déclenchement (44) pour retenir le mécanisme de libération (38) ; et
un moyen pour amortir le mouvement de l'organe de libération (38) ;
dans lequel, lorsque le mécanisme est activé, l'organe de déclenchement (44) libère l'organe de libération (38) et l'organe de libération (38) se déplace, sous l'influence de sa sollicitation élastique et à l'encontre de la résistance du moyen d'amortissement, de manière à libérer la seringue (4).

7. Dispositif d'injection selon la revendication 6, dans lequel le moyen d'amortissement comporte un fluide pour amortir le mouvement de l'organe de libération (38).

8. Dispositif d'injection selon la revendication 7, dans lequel le fluide d'amortissement est un liquide.

9. Dispositif d'injection selon l'une quelconque des revendications 6 à 8, dans lequel l'organe de libération (38) comporte un collier.

10. Dispositif d'injection selon la revendication 9, dans lequel le moyen d'amortissement est prévu entre le collier (38) et le boîtier.

11. Dispositif d'injection selon l'une quelconque des revendications 6 à 10, dans lequel l'organe de déclenchement (44) peut être déplacé par l'accouplement (32, 34) d'une position qui empêche le mouvement de l'organe de libération (38) dans une position qui permet le mouvement de l'organe de libération (38).

12. Dispositif d'injection selon l'une quelconque des revendications précédentes, dans lequel l'accouplement comprend des premier (32) et deuxième (34) éléments et le mécanisme de libération (38) est prévu pour désengager les premier et deuxième éléments (32, 34) pour libérer la seringue (4).

13. Dispositif d'injection selon la revendication 12, dans lequel le premier élément (32) comprend une paroi annulaire définissant un alésage central et le deuxième élément comporte des bras élastiques (42) qui s'engagent avec la paroi annulaire et qui sont capables de fléchir vers l'intérieur pour se désengager de la paroi annulaire et permettre au deuxième élément (34) de se déplacer par rapport au premier élément (32) à l'intérieur de l'alésage.

14. Dispositif d'injection selon la revendication 5, dans lequel :
le mécanisme de libération comporte une masse d'inertie (76) pouvant être déplacée avec l'accouplement (70, 72) et un organe de libération actionné par la masse d'inertie (76) pour libérer la seringue (4), de telle sorte que lorsque l'accouplement (70, 72) atteint ladite position prédéterminée par rapport au boîtier, la masse d'inertie continue de se déplacer indépendamment du mouvement de l'accouplement (70, 72), de manière à actionner l'organe de libération pour libérer la seringue (4).

15. Dispositif d'injection selon la revendication 14, dans lequel la masse d'inertie (76) est montée sur l'accouplement (70, 72) de manière à permettre un mouvement relatif de la masse d'inertie (76) dans la direction du mouvement de l'accouplement (70, 72) au cours de la décharge de la seringue (4).

16. Dispositif d'injection selon la revendication 15, dans lequel la masse d'inertie (76) est montée sur l'accouplement (70, 72) au moyen d'un filetage.

17. Dispositif d'injection selon la revendication 15 ou la revendication 16, comportant en outre une butée (80) qui s'engage avec la masse d'inertie pour empêcher un mouvement relatif de la masse d'inertie (76) dans la direction opposée à la direction de mouvement de l'accouplement (70, 72) au cours de la décharge de la seringue (4).

18. Dispositif d'injection selon la revendication 17, dans lequel la butée (80) est prévue sur l'accouplement (70, 72).

19. Dispositif d'injection selon l'une quelconque des revendications 14 à 18, dans lequel l'accouplement (70, 72) comprend des premier (70) et deuxième (72) éléments et l'organe de libération peut être déplacé par la masse d'inertie (76) pour désengager les premier (70) et deuxième (72) éléments pour libérer la seringue (4).

20. Dispositif d'injection selon la revendication 19, dans lequel l'organe de libération est intégré à la masse d'inertie (76) et relie les premier (70) et deuxième (72) éléments, de telle sorte qu'un mouvement relatif de la masse d'inertie (76) et de l'accouplement (70, 72) sépare les premier (70) et deuxième (72) éléments pour libérer la seringue (74).

21. Dispositif d'injection selon la revendication 20, dans lequel les premier (70) et deuxième (72) éléments sont pourvus de filetages coaxiaux et l'organe de libération est pourvu d'un filetage correspondant pour relier les premier et deuxième éléments d'accouplement (70, 72).
